# EUROPEAN PATENT SPECIFICATION

(11) **EP 1 299 124 B1**
(45) Date of publication and mention of the grant of the patent: **24.03.2010**
(21) Application number: 01984093.3
(22) Date of filing: 02.07.2001
(51) Int. Cl.: A61K 47/10, A61K 47/12, A61K 47/18, A61K 9/00

(54) **COMPOSITION FOR AQUEOUS STABILIZATION OF FAT-SOLUBLE VITAMINS**
ZUSAMMENSETZUNG ZUR STABILISIERUNG VON FETTLÖSLICHEN VITAMINEN IN WÄSSERIGER LÖSUNG
COMPOSITION SERVANT A STABILISER DES VITAMINES LIPOSOLUBLES DANS UNE SOLUTION AQUEUSE

(30) Priority: 04.07.2000 HU 0002561
(43) Date of publication of application: 09.04.2003
(73) Proprietor: Vitalitae Biotech GmbH, 5405 Baden-Dättwill AG (CH)
(72) Inventor: Bertha, András, 2092 Budakeszi (HU)
(74) Representative: Lantos, Mihaly
(86) International application number: PCT/HU2001/000073
(87) International publication number: WO 2002/002145

(56) References cited:
- WO-A-00/69999
- WO-A-98/23152
- CH-A- 388 536
- US-A- 3 252 864

## Description

The invention relates to an aqueous liquid composition comprising fat-soluble vitamins for human or veterinary applications, characterized by comprising a stabilizing solution having:
(i) an alcohol with 4 to 8 carbon atoms
(ii) 0.5 to 3 mass unit of amine or amide having 2 to 8 carbon atoms calculated for a unity mass of alcohol; and
(iii) 1.5 to 6 mass unit of carboxylic acid with 8 to 18 carbon atoms calculated for a unity mass of alcohol.
wherein the total amount of the stabilizing solution is at least 2 mass%, preferably lying between 2 and 6 mass %, and that of the vitamins between 20 and 40 mass % and to the use of such composition as an additive to the drinking water for feeding animals.

In the field of animal breeding the problem connected with feeding fat-soluble vitamins has long been known. In contrast to water-soluble vitamins such vitamins can not be fed in drinking water but only in feed premixes. Such a dosage is difficult and inaccurate since one has to provide and distribute the fat-soluble vitamins and/or pro-vitamins in the daily feed with an accuracy of a few milligrams. A typical example for such a feeding is the composition described in U.S. patent 3,252,864, which comprises in addition to the fat-soluble vitamins a specific antioxidant and an amine-containing group. These compositions are mixed to the solid or semi-solid feed of animals to ensure the required consumption of fat-soluble vitamins.

The portioning of vitamins in drinking water is accurate, and the animals can take the available active materials always according to their needs. Of that reasons it would be preferable if the animals could obtain their daily or periodic need for vitamins non-soluble in water by drinking, however, owing to the non-soluble nature of such vitamins in water this could not have been solved so far.

There have been a few number of experiments for making such water insoluble vitamins and pro-vitamins soluble in water, e.g. those described in the book of Gy. Kedvessy: "Gyógyszertechnológia (Technology of Pharmaceuticals)", Budapest, 1971. Such experiments describe applications in emulsions only. Here, the fat-soluble vitamins and/or pro-vitamins are dissolved in respective plant oils according to the required purpose or solubility,then by using different type emulsifying agents or surface active materials an aqueous emulsion is made. This method results in a vitamin concentrate. Such an emulsion product comprises one or more vitamins and/or pro-vitamins, which can be added to the drinking water of the animals following an appropriate dilution.

Such emulsions have not obtained a wide range of applications because stability problems have arose in case of higher dilution. Such problems can be read in the publication of E. Juhász: "Felületaktív anyagok zsebkönyve" (Pocket book of surfactants), Budapest, 1979. As a further consequence the problem emerges that the separated oily phase gets deposited on the wall of the drinking apparatus, it clogs the valves and can cause problems in the function.

Emulsion concentrates cannot be diluted to several thousand or several tens of thousands times, during the dilution the oily phase gets separated from the wet phase, thus the task of secure dosage of fat-soluble vitamins to drinking water has not been solved so far.

In this way, the currently known methods cannot solve the problem of preparing a stable pharmaceutical preparation for fat-soluble vitamins and/or pro-vitamins in an aqueous system, which could be used in animal breeding by being safely added to the drinking water.

The invention covers the full supply of all vitamins and selenium, and given cases with the addition of suitable amount and type of amino-acids, in the drinking water of all breeding stock and animals bred for sport and other purposes by using a composition that constitutes a micro-emulsion and/or macro-molecular disperse system.

The above described application of this composition has, among other things, the objective to prevent the widely known damage of vitamins, selenium and predetermined amino-acids that take place during mixing and storage with granular feed and polivalent minerals. This is proven with the unexpected surprising result which will be described in the first example.

Surveying upon evaluating the requirements it has become apparent that for the sake of safe storage, stability, use and dosage preparations are required which besides the preferred presence of water can safely provide the required amounts (dosages) of the subject fat-soluble vitamins and/or pro-vitamins.

In addition to the presence of water such products or preparations must be clear like water, clean and free from sediments, and such state should be retained through several years.

In given cases e.g. in animal breeding such preparations should remain stable and suitably homogenous upon being diluted by water for drinking.

During our research we have arrived at the surprising recognition that a liquid and aqueous preparation is obtained that satisfies all the above requirements and comprises fat-soluble vitamins and/or pro-vitamins if the system is stabilized by a composition that comprises:
(i) an alcohol with 4 to 8 carbon atoms
(ii) 0.5 to 3 mass unit of amine or amide having 2 to 8 carbon atoms calculated for a unity mass of alcohol; and/or
(iii) 1.5 to 6 mass unit of carboxylic acid with 8 to 18 carbon atoms calculated for a unity mass of alcohol.

For example the stabilizing composition of a total mass of 1000 g comprises:
- alcohol with 4 to 8 carbon atoms: 1,3- octane diol: 10 g
- propanol diamine or heptanol amine: 10 g
- lauric acid or stearic acid: 30 g.

In addition to the composition of altogether 50 g i.e. 5 % the amount of 1000 g comprises vitamins and pro-vitamins in a total amount of 380 g and 570 g of ion-exchanged water.

The so obtained composition of 1000 g is substantially a master solution which can be diluted in any required amount. The upper limit of dilution is limited by practical aspects, i.e. that in the daily consumed drinking water of the animals should comprise the daily vitamin demand.

In the preparation the lower limit of the composition is defined by the value required for stabilizing the vitamin mass, and this limit is in general between 1 and 2 % in case if the total vitamin mass is 38 %. The upper limit is defined by economic consideration only.

A usual dilution of the preparation is about 1 to 100. The dilution can simply be carried out by adding the composition to the drinking water of the animals in the appropriate dose. The animals obtain drinking water in harmony with their daily consumption of feed.

The composition according to the invention is distinguished basically from the earlier known aqueous compositions of fat-soluble vitamins and/or pro-vitamins by not having a single surfactant or emulgator component.

It is also surprising that the admixture of this composition to water and to a fat-soluble vitamin and/or pro-vitamin after being dissolved in an organic phase, a completely clear and transparent liquid is obtained, that preserves stability through several years. Up to the present, we have samples stored for more than 2 years and during this period nothing has changed.

The result according to the invention is obtained if the composition is mixed in cold state to the aqueous and organic phases.

Based on the above, without giving a too theoretic explanation, it can be supposed that in the common presence of water and the organic phase (in which vitamins and/or pro-vitamins are available in dissolved state) the respective components of the composition enter together and/or with water and/or with the organic phase with a chemical reaction having a nature not yet completely discovered, and as a result the aqueous and oily (organic) components that in their original state do not mix, a continuous phase transition is formed in a molecular or closely molecular level.

The alcohols, amides and/or carboxylic acids used in the composition according to the invention can have linear, branching or ring carbon chain and in given cases they can have unsaturated carbon skeleton with the exception of aromatic skeleton.

The alcohols used can be single- or multi-valent alcohols, however, the use of multi-valent (multi-hydric) alcohols is preferred.

In a similar way the carboxylic acid can be single- or multivalent, wherein the first type is preferred.

Also, the amines and amides can be single- or multi-valent, including in the latter group compounds being partially aminated.

It will be apparent for the man skilled in the art that the alcohols, carboxylic acids, amines and amides can equally be individual compounds or mixtures of a multiplicity of such compounds i.e. several types of alcohols and carboxylic acids can be used together. It will also be apparent that the aforementioned components can be used in any form of isomers, e.g. as geometric or optical isomers or as a mixture of the solutions of such isomers presented in any required ratio.

The composition according to the invention can comprise in addition to the selected blend of fat soluble vitamins and/pro-vitamins formulation agents e.g. for improving consistency and flavor. These agents can be added in the usual, conventional concentrations, as it is apparent to the man skilled in the art, to be present in the composition or preparation according to the invention.

Depending on the actual field of use the water used in the stabilized liquid product can be tap water or distilled water or, in case of using an aseptic method, it can also be sterilized distilled water.

In the product according to the invention when used in animal breeding the hardness of the tap water in the product does not exert a negative influence on either the quality or the stability.

The stabilized liquid products according to the invention are prepared by the mixing of the components. Owing to the fact that this is a very simple process, the formulation for obtaining a liquid pharmaceutical can be made in a plant equipped for liquid technology.

The vitamins and/or pro-vitamins not-soluble in water are solved in the oily phase, then they are mixed with the amount of water determined for the particular product, and under continued mixing the required amounts of additives are added.

By means of the composition according to the invention the fat-soluble vitamins and other materials are brought in a wet phase, and they are given to drinking water of the animals, wherein the amount of the active material is selected to correspond to the water consumption of the particular animals and to their intended way of utilization.

The additives required to the formulation of the product can be admixed at any phase of the liquid technology.

The order of the steps can be different from the one disclosed, and the individual components of the composition can be added individually, however, such a method is more labor intensive.

According to a further aspect of the invention, the composition can be used for stabilizing vitamins and/or pro-vitamins not-soluble in water, and the use of the stabilized liquid pharmaceutical product in animal breeding, in the field of veterinary and for the replacing premixes in the animal feed.

A further advantage of the composition according to the invention lies in that the composition is compatible with available animal breeding technologies, e.g. they can be used without any specific adjustment with existing automatic drinking equipment. Thus the vitamin concentrates made according to the invention can be directly fed in a drug feeder coupled to the automatic drinking equipment.

Following the adjustment of the drug feeder according to actual requirements, the vitamin concentrate according to the invention will be diluted by the required extent and will arrive to the target organizations, in the present case to the poultry described in the following examples (baby chickens, turkeys, baby geese).

A further advantage comes from the long stability of the products made according to the invention without any decrease in the concentration of the active ingredients. The products can be diluted by normal tap water without any decrease in quality, furthermore there will be no contaminating deposits on the walls of the drinking system.

A further advantage of the invention lies in that the active ingredients (i.e. vitamins and pro-vitamins) are available for the target animals in the most appropriate form (i.e. in their drinking water) without any limitation regarding time and place of availability.

The invention will now be described in connection with examples.

### Example 1:

The examinations were directed to establish the differences between the conventional addition of the required amount of vitamins first in the premix and then to the feed, and their addition according to the invention in a dissolved state to the drinking water.

In the control group treated in the conventional way the following vitamins were added to each kg of the feed:

| | |
|---|---|
| Vitamin A | 15 000 IU |
| Vitamin D (cholecalcipherol) | 3 500 IU |
| Vitamin E | 60 mg |
| Vitamin K(3) | 17 mg |
| Vitamin C | 20 mg |
| Vitamin U | 200 mg |
| Vitamins B (1, 2, 6) | each 15 mg |
| Vitamin B (12) | 0,1 mg |
| Biotin | 0,1 mg |
| Carnitine | 1 mg |
| Selenium | 0,3 mg |
| Nicotinic acid | 60 mg |
| Dambose | 20 mg |
| Ca-pantothenate | 20 mg |
| folic acid | 0,8 mg |
| cholin chloride | 600 mg |

In case of the animals treated according to the invention, a concentrate was added to the drinking water associated with the consumed mass of feed in a 1 to 100 dilution, wherein 1000 g of the concentrate comprised:
- 10g octane diol;
- 10g propanol diamine; and
- 30g lauric acid.

In addition to the composition having the mass of 50 g representing 5 %, the total amount of 1000 g comprises vitamins and pro-vitamins in an amount of 380 g comprising the same components as in case as in the control group, furthermore 570 g of ion-exchanged water.

The fat-soluble vitamins were made water-soluble by the 5 % composition, and an appropriate stock solution was prepared by adding the water-soluble vitamins, and the stock solution was diluted in the above described ratio and was fed in the automatic drinking system through an automatic feeder, and the experimental animal drank such a water.

The treatment was carried out both in the control and the experimental groups with respective 17000 broiler chicken through 40 days until the animals were butchered. The animals in both groups consumed the same feed during the cramming period.

At the end of the cramming period, the following results were obtained: the average butchered weight of the experimental animals was 2.13 kg, and the average specific feed consumption was 1.97 feed/kg.

The average butchered weight of the animals in the control group was 1.98 kg, and the average specific feed consumption was 2.13 feed/kg.

The vitamins supplied with the drinking water increased the average butchered weight by 9.35 % and decreased the specific feed demand associated with 1 kg increase in weight by 10.9 %.

Beside the above results the fact should be emphasized that the animals in the experimental group did not obtain any antibiotic or other drug treatment during the breeding period, while in the control group conventional drug treatment was applied.

During the experiment in the control group 4.7 % of the animals died owing to intestinal inflammation, while in the experimental group the average death rate was 0.4 %.

The animals in both group obtained all compulsory vaccination according to the conventional vaccination program.

### Example 2

Both the control and the experimental group comprised respective 170 male and 640 female geese of the Landes type, and the treatment followed the composition described in Example 1 with the difference that the amount of vitamins was complemented with beta-carotene in an amount of 4 microgram per consumed feed portion. The experiment took place in cycles of 3 weeks with the addition of vitamins including beta-carotene and 1 week of intermission (no vitamins added). The control group obtained a similar cyclic treatment, however, the vitamins were admixed to the premix and not the drinking water.

In a laying cycle the average number of eggs lain by a hen-bird was 34, while the average number of hatched eggs were 20.4.

The animals which drank the water comprising the composition according to the invention the average number of eggs lain was 52, and the hatching rate has never decreased below 90 %.

Based on these egg production and hatching numbers it has been proven that the use of fat-soluble vitamins and/or pro-vitamins (in the present case beta-carotene) applied in aqueous phase constitute an efficient method.

### Example 3

A composition similar to the one described in Example 1 was used, however, the components of the vitamins added for 1000 g of feed was the following, which was admixed to the feed of the control group.

| | |
|---|---|
| Vitamin A | 8 000 IU |
| Vitamin D (cholecalcipherol) | 2 000 IU |
| Vitamin E | 30 mg |
| Vitamin K(3) | 12 mg |
| Vitamin B (1) | 2 mg |
| Vitamin B (2) | 5 mg |
| Vitamins B (6) | 3 mg |
| Vitamin B (12) | 0.012 mg |
| Ca-pantothenate | 20 mg |
| Nicotinic acid | 22 mg |
| Folic acid | 1mg |
| Biotin | 0,2 mg |
| cholin chloride | 350 mg |
| Vitamin C | 30 mg |
| Vitamin U | 200 mg |
| Carnitine | 3 mg |

In case of the animal group treated according to the invention the drinking water provided in accordance with the feed consumed comprised a concentrate diluted in a 1:100 ratio, wherein 1000 g mass of the concentrate comprised:
- 10g octane diol;
- 10g heptanol amine; and
- 30g stearil acid.

In addition to the composition of 50 g i.e. 5 % the 1000 g concentrate comprised additionally vitamins and pro-vitamins of 380 g being composed from the same components as the ones used in the control group, furthermore 570 g of ion-exchanged water.

The addition of the composition of 50 g made the fat-soluble vitamins water-soluble, then the water-soluble vitamins were added and a stock solution was thereby made and this was fed in the aforementioned dilution through an automatic drug feeder into the automatic drinking system, and the animals drank this liquid.

The experiments were carried out on Cherry-Wery type ducks consisting of a fowl of 42000 ducks in the last week of the breeding period, and the control group comprised the same number and type of ducks, that obtained vitamins added and mixed to the feed.

In the experimental group the weight increase of the ducks exceeded in average by 400 g that of the control group.

This example also supports that the drinking of fat-soluble vitamins made water soluble according to the invention and added to the drinking water results in a more efficient utilization and take up of the vitamins compared to the conventional addition of the same vitamins mixed to the feed in the form of a premix.

### Example 4.

In both groups 18000 broiler chickens in the age of 5 weeks, respectively received the composition described in Example 1 through 1 week, the only difference lied in that the amount of vitamin K(3) was 19 mg for a feed of 1000 g. In the experimental group the drinking occurred through a drip feeder drinking machine.

The animals were slaughtered and qualified on the same day.

In the slaughtering process the disqualification rate in the control group due to bleedings in the wing and leg muscles was 50.7 %, while in the experimental group which drank also vitamin K(3) this value was only 23 %.

As a result of drinking of the composition according to the invention, the average slaughtering weight of the experimental group was by 40 g higher than that of the control group.

### Example 5.

The effects of the composition described in Example 1 and diluted in a ratio of 1:30 were examined on beeves, more particularly how the continuous feeding of small portions of the composition influences the health status and production level of milk producing beeves.

In the farm the keeping of the beasts was free, with increasing litter, and the stalls were closed from three sides. The feeding was complete and mono-dietary, group wise improved. The feeding occurred three times a day, practically ad libitum, a combined mixer and chopper feeder cart of the type "SEKO" was used. As a mass feed the cows obtained corn silage, lucern-hay and meadow-hay, furthermore the dairy herd obtained a forage assisting milk formation. The non-pregnant beeves obtained only corn silage and meadow-hay. For producing 1 liter milk 0.4 kg of milk-forming feed was used, when calculated according to the milk production of the individual groups used.

The milking occurred twice a day by means of a milking apparatus equipped with a chaplet.

The following groups were included in the experiments, each having 40 beeves:
- non-pregnant cows
- pregnant heifers in a status of pregnancy over 7 months
- group of receiving cows (cows being closely after the second delivery)
- group of receiving heifers (heifers being closely after the first delivery).

Starting from December 1, 2000 the 30 times diluted solution of 6 ml of the cited stock solution was uniformly sprinkled in every morning on the chopped and homogenized feed of the experimental groups by means of a garden sprinkler.

### The results of the examination:

The data of the milk production of the farm are summarized for five months before starting the experimental feeding (i.e. December 01, 2000).

**Table 1.**

| Period | Daily average number of cows | Daily average number of cows milked | Milking average l/milked-cow/day | Farm average l/total of the cows/day |
|---|---|---|---|---|
| July 2000 | 207.7 | 165.7 | 24.99 | 19.94 |
| August 2000 | 220.7 | 189.5 | 24.78 | 21.28 |
| September 2000 | 218.6 | 192.5 | 26.92 | 23.70 |
| October 2000 | 220.8 | 191.5 | 26.58 | 23.06 |
| November 2000 | 222.2 | 183.7 | 27.27 | 22.55 |
| December 2000 | 230.8 | 194.2 | 26.64 | 22.41 |
| January 2001 | 237.4 | 201.3 | 26.65 | 22.50 |
| February 2001 | 245.8 | 213.4 | 27.73 | 24.07 |
| March 2001 | 246.1 | 216.2 | 29.27 | 25.71 |

**Table 2: data on the occurrence of the caul retention following delivery.**

| Period | Number of deliveries | Number of caul retentions (number) | Proportion of caul retention (%) |
|---|---|---|---|
| September 2000 | 16 | 5 | 31 |
| October 2000 | 18 | 6 | 33 |
| November 2000 | 21 | 9 | 42 |
| December 2000 | 20 | 12 | 60 |
| January 2001 | 29 | 5 | 17 |
| February 2001 | 13 | 2 | 15 |
| March 2001 | 15 | 1 | 6 |

Data showing the duration of the involution and of the service period.

The duration of the involution has shortened by 4-5 days relative to the earlier periods. The insemination carried out within 60 days from the delivery was 30-35 % prior to the experimental feeding, in the month of March this ratio has reached the value of 72%.

The health status of the udder and data on the number of somatic cells. According to our findings the time required for the healing of animals suffering from mastitits has shortened following the experimental feeding, because it had lasted 8 to 10 days prior to the addition of the vitamins, while thereafter it became 4-5 days long.

The data on the number of somatic cells were obtained by comparing an other similar farm of the enterprise in Sárpilis which has similar size and the animals are kept and fed in a similar way as in Kajmád, where the treatment took place.

**Table 3.**

| Period | Farm in Sárpilis Thousand/cm³ | Farm in Kajmád Thousand/cm³ |
|---|---|---|
| January 2001 | 520 | 341 |
| February 2001 | 539 | 407 |
| March 2001 | 619 | 356 |

The general status of the animals, data on feed consumption.

As a result of the experimental feeding negative changes in the health status of the animals have not been experienced. The feed consumption data are shown in the following Table 4.

**Table 4.**

| Specific feed consumption for 1 liter milk produced | | | | |
|---|---|---|---|---|
| Definition of the feed | 1998 | 1999 | 2000 | 2001 I-III. months |
| forage assisting milk formation (kg) | 0.41 | 0.44 | 0.40 | 0.40 |
| corn silage | 1.21 | 1.47 | 1.09 | 0.98 |
| lucem-hay | 0.52 | 0.46 | 0.37 | 0.15* |
| hay | 0,19 | 0,09 | 0,03 | 0,04 |

| | | | | |
|---|---|---|---|---|
| * owing to the draught in the year 2000, the availability of lucern-hay was limited at the beeves farm. | | | | |

From the above examples it can be seen that the vitamin concentrates comprising fat-soluble vitamins and other active materials made according to the invention water-soluble, are suitable for use in breeding and cramming poultry, in quality meet production, in milk production, for facilitating the biology of multiplication and in several other fields. The bringing of vitamins in the organization of the animals is much more efficient and successful by using the suggested drinking technology as if the same vitamins were given in a form added to the premix.

The use of the composition according to the invention can be preferable in certain indications in the human field. In case of a healthy nutrition the bringing of fat-soluble vitamins in the human organization is not problematic, however, there are situations where unbalance takes place and the ingestion of these vitamins by a fat-free carrier is highly required. Such a case is e.g. the rehabilitation of a weakened and ill organization, and in case of serious diets the maintaining of equilibrium. The composition according to the invention can be mixed simply to normal drinking water, in an efficient concentration it does not have an unpleasant taste, and the ingestion of vitamins does not require the processing of fats that would otherwise be difficult to digest. The composition according to the invention can thus be used preferably in human applications.

## Claims

1. An aqueous liquid composition comprising fat-soluble vitamins for human or veterinary applications, **characterized by** comprising a stabilizing solution having:
(i) an alcohol with 4 to 8 carbon atoms
(ii) 0.5 to 3 mass unit of amine or amide having 2 to 8 carbon atoms calculated for a unity mass of alcohol; and
(iii) 1.5 to 6 mass unit of carboxylic acid with 8 to 18 carbon atoms calculated for a unity mass of alcohol;
wherein the total amount of the stabilizing solution is at least 2 mass% and that of the vitamins is between 20 and 40 mass%.

2. The composition as claimed in claim 1, **characterized in that** the stabilizing solution comprises a single or multi-valent alcohol as component (i).

3. The composition as claimed in claim 2, **characterized in that** the stabilizing solution comprises octane diol as component (i).

4. The composition as claimed in claim 1, **characterized in that** the stabilizing solution comprises a monoamine or diamine as component (ii).

5. The composition as claimed in claim 4, **characterized in that** the stabilizing solution comprises heptanol amine or propanol diamine as component (ii).

6. The composition as claimed in claim 1, **characterized in that** the stabilizing solution comprises a single or multi-valent carboxylic acid as component (iii).

7. The composition as claimed in claim 1, **characterized in that** the stabilizing solution comprises lauric acid or stearic acid as component (iii).

8. The composition as claimed in claim 1, **characterized by** comprising additionally at least one fat-soluble pro-vitamin.

9. Use of the composition of claim 1 as an additive to the drinking water for feeding animals, **characterized in that** said composition is added to the drinking water of the animals in a substantial dilution.

10. The use as claimed in claim 9, **characterized in that** the range of said dilution lies between 1 to 30 and 1 to 100.

## Patentansprüche

1. Eine wässrige flüssige Zusammensetzung enthaltend fettlösliche Vitamine für menschliche oder tierische Verzehrung, **gekennzeichnet durch** einen Gehalt an einer stabilisierenden Lösung enthaltend
(i) ein Alkohol mit 4 bis 8 Kohlenstoffatomen,
(ii) 0,5 bis 3 Gewichtseinheiten von einem Amin oder Amid mit 2 bis 8 Kohlenstoffatomen, gerechnet für eine Gewichtseinheit vom Alkohol, und
(iii) 1,5 bis 6 Gewichtseinheiten von einem Karbonsäure mit 8 bis 18 Kohlenstoffatomen, gerechnet für eine Gewichtseinheit vom Alkohol;
wobei die Gesamtmenge der stabilisierenden Lösung mindestens 2 Gewicht% und die der Vitamine zwischen 20 und 40 Gewichts% betragen.

2. Zusammensetzung nach Anspruch 1, **dadurch gekennzeichnet, dass** die stabilisierende Lösung ein ein- oder mehrwertiges Alkohol als Komponente (i) enthält.

3. Zusammensetzung nach Anspruch 2, **dadurch gekennzeichnet, dass** die stabilisierende Lösung Oktandiol als Komponente (i) enthält.

4. Zusammensetzung nach Anspruch 1, **dadurch gekennzeichnet, dass** die stabilisierende Lösung ein Monoamin oder Diamin als Komponente (ii) enthält.

5. Zusammensetzung nach Anspruch 4, **dadurch gekennzeichnet, dass** die stabilisierende Lösung Heptanolamin oder Propanoldiamin als Komponente (ii) enthält.

6. Zusammensetzung nach Anspruch 1, **dadurch gekennzeichnet, dass** die stabilisierende Lösung eine ein- oder mehrwertige Karbonsäure als Komponente (iii) enthält.

7. Zusammensetzung nach Anspruch 1, **dadurch gekennzeichnet, dass** die stabilisierende Lösung Laurinsäure oder Stearinsäure als Komponente (iii) enthält.

8. Zusammensetzung nach Anspruch 1, **dadurch gekennzeichnet, dass** sie weiterhin mindestens ein fettlösliches Provitamin enthält.

9. Verwendung der Zusammensetzung nach Anspruch 1 als Zusatzstoff für Trinkwasser zur Fütterung von Tieren, **dadurch gekennzeichnet, dass** die Zusammensetzung in einer substantiellen Verdünnung zum Trinkwasser von Tieren zugegeben wird.

10. Die Verwendung nach Anspruch 9, **dadurch gekennzeichnet, dass** das Bereich der Verdünnung zwischen 1 bis 30 und 1 bis 100 liegt.

## Revendications

1. Une composition liquide aqueuse pour application humaine ou vétérinaire comprenant des vitamines solubles dans la matière grasse, **caractérisée en ce qu'**elle comprend une solution stabilisante contenant :
(i) un alcool avec 4 à 8 atomes de carbone
(ii) 0.5 à 3 unité de masse d'une amine ou d'un amide avec 2 à 8 atomes de carbone, calculé pour une unité de masse de l'alcool; et
(iii) 1.5 à 6 unité de masse d'un acide carboxylique comprenant 8 à 18 atomes de carbone, calculé pour une unité de masse de l'alcool ;
où la quantité totale de la solution stabilisante est au moins 2 % massique et celle des vitamines est entre 20 et 40 % massique.

2. La composition selon la revendication 1, **caractérisée en ce que** la solution stabilisante comprend un alcool mono- ou multivalent en tant que composant (i).

3. La composition selon la revendication 2, **caractérisée en ce que** la solution stabilisante comprend octanediol en tant que composant (i).

4. La composition selon la revendication 1, **caractérisée en ce que** la solution stabilisante comprend une monoamine ou une diamine en tant que composant (ii).

5. La composition selon la revendication 4, **caractérisée en ce que** la solution stabilisante comprend heptanolamine ou propanoldiamine en tant que composant (i).

6. La composition selon la revendication 1, **caractérisée en ce que** la solution stabilisante comprend un acide carboxylique mono- ou multivalent en tant que composant (iii).

7. La composition selon la revendication 1, **caractérisée en ce que** la solution stabilisante comprend l'acide laurique ou l'acide stéarique en tant que composant (iii).

8. La composition selon la revendication 1, **caractérisée en ce qu'**elle comprend également au moins une pro-vitamine soluble dans la matière grasse.

9. L'utilisation de la composition selon la revendication 1 comme une additive à l'eau potable pour alimenter des animaux, **caractérisée en ce que** la composition est ajoutée à l'eau potable des animaux en une dilution considérable.

10. L'utilisation selon la revendication 9, **caractérisé en ce que** la gamme de la dilution se trouve entre 1 à 30 et 1 à 100.
